# EUROPEAN PATENT APPLICATION

(11) **EP 0 535 530 A1**
(43) Date of publication of application: **07.04.1993**
(21) Application number: 92116339.0
(22) Date of filing: 24.09.1992
(51) Int. Cl.: A61L 15/44, A61M 35/00, A61K 33/24, A61N 1/18

(54) **Extended-life drug filled patch**

(30) Priority: 01.10.1991 US 769601
(71) Applicant: Becton Dickinson and Company, Franklin Lakes, New Jersey 07417-1880 (US)
(72) Inventor: Haynes, John L., Chapel Hill, North Carolina 27514 (US)
(74) Representative: von Kreisler, Alek, Dipl.-Chem.

(57) **Abstract**

The invention discloses extended life drug filled transdermal patches and methods for preventing microbial degradation of a drug filled patch. The patches comprise a preservative amount of an antimicrobial metal to prevent microbial degradation of the drug.

## Description

### FIELD OF THE INVENTION

The invention is in the area of transdermal drug delivery. In particular the invention is in the area of transdermal drug delivery patches filled with drugs. More particularly, the invention is directed toward patches filled with drugs for transdermal delivery in which the shelf life is considerably long.

### BACKGROUND OF THE INVENTION

Transdermal drug delivery involves the continuous administration of therapeutic molecules through the skin. It has the advantage of maintaining constant drug plasma levels and improving patient compliance. In addition, compared to the oral route, losses in bioavailability due to first pass liver metabolism are reduced.

Transdermal drug delivery is typically divided into passive and active delivery. Passive delivery primarily being defined as the application of a particular drug with various enhancers and so forth to the skin, without electrical current. Active transdermal drug delivery, however, is typically referred to as iontophoresis (or ion transfer) and involves the migration of ions when an electrical current is passed through a solution containing ionized species. Also included in active transdermal delivery is electroosmosis, phonophoresis, and the like. With iontophoresis, drugs in their ionic form can be driven through the skin into the body with a small current.

In both active and passive transdermal delivery systems, there is a need to provide the ultimate user of such devices with drugs suitable for use in either an iontophoretic system or a passive system.

Typically, to extend shelf life of the various systems employing drugs, chemical preservatives, irradiation, autoclaving, sterile fill procedures, and the like are used. However, prevention of microbial contamination is still an issue. One aspect of providing an extended shelf life of a drug for use in a passive or active transdermal delivery system is to prevent microbial action within the patch.

The present invention overcomes microbial contamination problems associated with active or passive systems employing drugs by providing a means for extending the shelf life of drug filled patches.

### SUMMARY OF THE INVENTION

The present invention provides a drug filled transdermal patch comprising a preservative amount of an antimicrobial metal.

Methods for preventing microbial degradation of a drug filled patch which comprises adding a preservative amount of an antimicrobial metal are also provided.

The use of antimicrobial metals in patches of the present invention provides numerous advantages. In particular, the use of antimicrobial metals as a preservative eliminates the use of the generally employed chemical preservatives, irradiation, autoclaving, sterile fill procedures, and the like. By eliminating the addition of chemical preservatives the addition of a second agent to the patch is eliminated. In addition, autoclaving and various sterilizing procedures are eliminated, as well as diverting the potential damage to the drug by use of such procedures. In a typical iontophoretic drug delivery system, an oligodynamic metal can be used as the anode in drug delivery. Thus, in an iontophoretic delivery system, the use of an oligodynamic metal provides the benefits of extended shelf life and antimicrobial activity. In addition, the use of oligodynamic metals eliminates the addition of other agents to the drug patch and also allows the electrode to double as a preservative.

As used in this document, "a preservative amount" is that amount that is antimicrobial to the extent that it extends the shelf life of the drug filled transdermal delivery patch beyond the shelf life without the preservative amount. The extended shelf life can be due to either inhibition of growth or destruction effects of the preservative amount of antimicrobial metals. "Antimicrobial metal" refers to metal capable of extending the shelf life of a drug filled patch. Therefore, these metals include pure metals, alloys, oxides thereof, salts thereof, activated forms thereof, and the like. "Patch" refers to the spectrum of means for containing drugs for transdermal delivery. Therefore, the term patch includes those for passive and active delivery and includes but is not limited to bandages, prefilled passive drug delivery patches, prefilled iontophoretic drug delivery devices, prefilled active drug delivery patches, and reusable iontophoretic drug delivery devices comprising a drug reservoir that is reusable or refillable.

### DETAILED DESCRIPTION OF THE INVENTION

The typical transdermal drug delivery system involves the use of a drug filled container, or a container capable of being filled with drug prior to use. Therefore, the typical transdermal drug delivery system involves patches filled with drug intended for one time use, or devices for containing drug filled patches for which the device is designed to be reused by using disposable drug filled patches.

The typical iontophoretic system involves the use of either a device capable of reusing drug filled patches or containers, or involves iontophoretic devices prefilled with drug in a reservoir, in which the entire device is disposed after drug delivery. With any system employed (e.g., passive or active), the present invention provides a transdermal drug delivery patch filled with drug that has enhanced shelf life. Suitable patch-like devices for use with the present invention include bandage-type devices as disclosed by M. Seiderman in U. S. Patent 4,767,401, patches disclosed by D. Sibalis in U. S. Patent 4,921,475, and disposable patches as disclosed by D. Sibalis in U. S. Patent 4,856,188. Suitable iontophoretic devices include those disclosed by J. Phipps et al in U. S. Patent 4,744,787 and D. Sibalis as disclosed in U. S. Patent 4,808,152.

Drug filled transdermal patches may contain products which are nutrient to microbes. Such products include but are not limited to peptides and proteins such as insulin, calcitonin, albumin, and the like.

Besides peptides and proteins that are feed for various microorganisms, breakdown products of peptides and proteins are also a problem. In addition, recombinant peptides and protein can have the additional problem of host protein contaninants that can be a growth source for microorganisms.

Microorganisms that are known to be susceptible to antimicrobial metals such as oligodynamic metals include both anaerobic and aerobic microorganisms. Examples include bacteria, protozoa, fungus, and viruses. Examples of specific microorganisms include Streptococcus, Enterobacter, Pseudomonas, Staphylococcus, Escheria, Proteus, Clostridia, and Candida. Specific microorganisms include Streptococcus pyogenes, Enterobacter cloacae, Pseudomonas aeruginosa, Staphylococcus aureus, Escherichia coli, Proteus vulgaris, Staphylococcus saprophyticas, Streptococcus faecalis, Klebsiella pneumoniae, and Candida albacans.

Silver is a preferred oligodynamic antimicrobial metal. Microorganisms that are known to be susceptible to silver include both anaerobic and aerobic microorganisms. Examples include bacteria, protozoa, fungus, and viruses. Examples of specific microorganisms include Streptococcus, Enterobacter, Pseudomonas, Staphylococcus, Escheria, Proteus, Clostridia, and Candida. Specific microorganisms include Streptococcus pyogenes, Enterobacter cloacae, Pseudomonas aeruginosa, Staphylococcus aureus, Escherichia coli, and Proteus vulgaris.

Silver, gold, platinum, lead, palladium, and copper are examples of oligodynamically active metals suitable for use in practicing the invention. Examples of such metals used in combination are metallic silver and metallic platinum, and metallic silver and metallic palladium. I.B. Romans, "Oligodynamic Metals" Chapter 24 and "Silver Compounds" Chapter 28 in Disinfection, Sterilization and Preservation, by C. A. Lawrence and S. S. Block, ed., Lea and Fibiger (1968) and U.S. Patent 4,886,505, both incorporated herein by reference, provide details of oligodynamic metals, their use, and their activities.

Silver is generally employed as an electrode in iontophoretic devices and is readily activated to produce preservative amounts of oligodynamic metals. Without activation, silver is not in itself antimicrobial. Thus, although activation of metals occurs during iontophoretic drug delivery, the present invention provides activation during storage (i.e., before iontophoretic delivery), thereby extending the shelf life of drug filled patches prior to use. An easy means of activating silver is by forming a salt or exposure to a dissimilar metal. For example, slight chloridation can be obtained by including the commercially available silver/silver chloride as the electrode. Silver can be used in a variety of forms including a commercially pure form, metal form, or alloy form such as silver solder. Silver is a preferred metal for patches comprising metal in the form of an electrode.

Metals that activate silver preferably include those metals more electronegative, but include platinum, palladium, copper and zinc. Metal salts such as silver bromide, silver fluoride, silver iodide, silver chloride, silver nitrate, silver sulfate, silver alkylcarboxylate, silver sulphadiazine and silver arysulfonate can also be used as a form of oligodynamic metal.

Oxides and salts of the metals include silver acetate, silver benzoate, silver carbonate, silver citrate, silver chloride, silver iodide, silver oxide, silver sulfate, and chloroplatinic acid.

Methods for preventing microbial degradation of a drug filled patch comprise adding a preservative amount of an antimicrobial metal to a patch. Any of the metals referenced in this document that are added in a preservative amount, in addition to others, can be added to a patch that will ultimately be a drug filled patch, thus extending the life of the patch and preventing microbial degradation.

Although the invention has been described with respect to specific modifications, the details thereof are not to be construed as limitations, for it will be apparent that various equivalents, changes, and modifications may be resorted to without departing from the spirit and scope thereof, and it is understood that such equivalent embodiments are to be included therein.

## Claims

1. A drug filled patch comprising a preservative amount of an antimicrobial metal.

2. The drug filled patch of claim 1 in which the antimicrobial metal comprises a metallic salt, metallic oxide, metallic silver or metallic platinum.

3. The drug filled patch of claim 1 in which the antimicrobial metal comprises an oligodynamic metal.

4. The drug filled patch of claim 1 in which the metal is an electrode.

5. The drug filled patch of claim 4 in which the electrode is silver.

6. The drug filled patch of claim 5 which further comprises chloridation.

7. A method for preventing microbial degredation of a drug filled patch which comprises adding a preservative amount of an antimicrobial metal.

8. The method of claim 7 in which the metal comprises a metallic salt, metallic oxide, metallic silver or metallic platinum.

9. The method of claim 7 in which the metal comprises an oligodynamic metal.

10. The method of claim 7 in which the metal is in the form of an electrode.
